# EUROPÄISCHE PATENTANMELDUNG

(11) **EP 2 960 704 A1**
(43) Veröffentlichungstag der Anmeldung: **30.12.2015**
(21) Anmeldenummer: 15172223.8
(22) Anmeldetag: 16.06.2015
(51) Int. Cl.: G02B 21/08, G02B 21/12

(54) **BELEUCHTUNGSEINRICHTUNG EINES OPERATIONSMIKROSKOPS**

(30) Priorität: 26.06.2014 DE 102014212372
(71) Anmelder: Carl Zeiss Meditec AG, 07745 Jena (DE)
(72) Erfinder: Reimer, Peter, 73479 Ellwangen (DE); Liegel, Jürgen, 73447 Oberkochen (DE); Bausewein, Markus, 83624 Otterfing (DE)
(74) Vertreter: Carl Zeiss AG - Patentabteilung

(57) **Zusammenfassung**

Bei einer Beleuchtungseinrichtung (1), insbesondere eines Operationsmikroskops (10), umfassend eine Beleuchtungsoptik (3) mit einer optischen Achse (13) und einer Lichtquelle (2) zum Erzeugen wenigstens eines Beleuchtungsstrahlengangs (11) zum Beleuchten eines Objektfelds unter einem bestimmten Beleuchtungswinkel und mit einem Beobachtungsstrahlengang (12), wobei im Beleuchtungsstrahlengang mindestens ein Spiegel (4, 5) zum Umlenken von Licht der Lichtquelle (2) vorgesehen ist und der Spiegel (4, 5) entlang der optischen Achse (13) eine Längserstreckung aufweist, wobei ein Licht emittierender Flächenbereich der Lichtquelle (2) ohne bewegliche Bauteile senkrecht zur optischen Achse (13) variierbar (x) ist, ist zur Erzielung einer gewünschten Beleuchtung die Beleuchtungsoptik (3) derart ausgebildet, dass eine Beleuchtungspupille (16) innerhalb der Längserstreckung des Spiegels (4, 5) abgebildet wird.

## Beschreibung

Die Erfindung betrifft eine Beleuchtungseinrichtung, insbesondere eines Operationsmikroskops, nach dem Oberbegriff des Patentanspruchs 1.

Aus der EP 1 997 423 B1 ist ein Operationsmikroskop mit Beleuchtungseinrichtung bekannt mit einer Beleuchtungsoptik und einer Lichtquelle. Dabei kann mit einem ersten Beleuchtungsstrahlengang eine achsnahe bzw. koaxiale Beleuchtung und mit einem zweiten Beleuchtungsstrahlengang eine achsferne Beleuchtung erhalten werden. Dabei werden als Winkel zwischen einem Beobachtungsstrahlengang und Beleuchtungsstrahlengang beispielsweise 2° oder 6° gewählt. Mit einem derartigen Operationsmikroskop kann unter anderem eine Kataraktoperation durchgeführt werden. Weiterhin sind diese Operationsmikroskope bzw. deren Beleuchtungseinrichtungen derart ausgebildet, dass im zu untersuchenden Auge der an sich bekannte Redreflex, d.h. ein rotes Aufleuchten der Augenpupille bei achsnaher Beleuchtung, erzeugbar ist. Somit können an sich transparente Strukturen beispielsweise einer zu entfernenden Augenlinse dargestellt werden. Dies wird entweder mit einer Koaxialbeleuchtung oder einer einfacher aufgebauten seitlichen Beleuchtung unter beispielsweise 2° Einfallswinkel realisiert. Alternativ bzw. zusätzlich zu einer 2°-Beleuchtung kann auch ein größerer Beleuchtungswinkel, z. B. 6°, gefordert werden, um eine noch höhere Kontrastierung, d.h. Licht-/ Schattenverteilung, zu erzielen und insbesondere die Makula auf der Netzhaut zu schonen, wenn kein Redreflex erforderlich ist wie bei einer Hornhauttransplantation.

Weiterhin beschreibt die DE 10 2004 029 056 A1 eine Beleuchtungseinrichtung für ein Operationsmikroskop, das beispielsweise für Augenoperationen eingesetzt wird. Die Beleuchtungseinrichtung umfasst einen Spiegel zur Umlenkung von Licht, wobei sich der Spiegel entlang einer optischen Achse der Beleuchtungseinrichtung über einen Längenbereich erstreckt. Das bedeutet, dass die Spiegelebene zur optischen Achse geneigt ist und die Projektion der Spiegelebene auf die optische Achse im Querschnitt gesehen eine gewisse Längserstreckung aufweist. Die Beleuchtungseinrichtung verfügt über eine Lichtquelle, die aus einer Matrix insbesondere von organischen Leuchtdioden (OLED) gebildet sein kann. Je nach dem welche dieser Kleinstlichtquellen aktiviert ist, in einer Ebene senkrecht zu einer optischen Achse der Beleuchtungseinrichtung gesehen, könnten gewünschte Beleuchtungsgeometrien erhalten werden, also zum Beispiel eine 2°- oder eine 6°-Beleuchtung. Insbesondere sind zur Erzeugung unterschiedlicher Beleuchtungsgeometrien keine mechanischen Bauteile notwendig. Eine gleichmäßige Ausleuchtung eines Objektfeldes beziehungsweise die gezielte Ausleuchtung eines bestimmten Ausschnittes davon, also beispielsweise eines zu operierenden Auges, ist hierbei aber nicht immer gewährleistet.

Ausgehend von diesem Stand der Technik ist der Fachmann vor die Aufgabe gestellt, ein Operationsmikroskop bzw. dessen Beleuchtungseinrichtung dahingehend zu verbessern, dass eine vom Nutzer gewünschte Beleuchtung, insbesondere unterschiedliche Beleuchtungswinkel, in einfacher Weise realisierbar ist und eine homogene Beleuchtung erzielbar ist.

Diese Aufgabe wird durch eine Beleuchtungseinrichtung mit den Merkmalen des Patentanspruchs 1 sowie durch ein nachfolgend beschriebenes Operationsmikroskop gelöst.

In an sich bekannter Weise ist ein Licht emittierender Flächenbereich der Lichtquelle senkrecht zur optischen Achse der Beleuchtungsoptik ohne bewegliche Bauteile variierbar. Dabei ist unter Lichtquelle diejenige Einrichtung der Beleuchtungseinrichtung zu verstehen, von der Licht über eine Beleuchtungsoptik in das eigentliche Operationsmikroskop eingebracht wird. Somit kann bei geeigneter Wahl der Beleuchtungsoptik ein Winkel, unter dem ein Beleuchtungsstrahlengang, der von diesem Flächenbereich ausgeht und beispielsweise auf ein Patientenauge einfällt, relativ zum Beobachtungsstrahlengang verändert werden. Es ist dabei ersichtlich, dass dieser Flächenbereich der Lichtquelle nicht nur in beispielsweise einer ersten x-Richtung senkrecht zur optischen Achse der Beleuchtungsoptik variierbar sein kann sondern zusätzlich oder alternativ auch in einer zur ersten x-Richtung senkrechten y-Richtung in einer Ebene senkrecht zur optischen Achse der Beleuchtungsoptik. Beispielsweise wie nachstehend näher beschrieben können mehrere separat zu steuernde Licht emittierende Flächen in einer Lichtquelle relativ nahe zueinander angeordnet sein, so dass je nach Anzahl, Intensität und Position der aktivierten Licht emittierenden Flächen ein Flächenschwerpunkt der spezifischen Lichtausstrahlung der Lichtquelle sich zumindest in einer Richtung senkrecht zur optischen Achse der Beleuchtungsoptik ändert. Die spezifische Lichtausstrahlung ist dabei die Lichtleistung pro Fläche der Lichtquelle, die in den gesamten Raumwinkel abgegeben wird. Insbesondere kann somit auf bewegliche Bauteile wie beispielsweise mechanische Blenden verzichtet werden. Ebenso ist es ermöglicht, dass je nach Wahl des Licht emittierenden Flächenbereichs praktisch nur genau derjenige Beleuchtungsstrahlengang erzeugbar ist, der gewünscht ist. Somit kann eine überflüssige Lichterzeugung, die beispielsweise zu einem unerwünschten Wärmeeintrag führen könnte, vermieden werden.

Weiterhin ist die Beleuchtungseinrichtung derart ausgebildet, dass die Beleuchtungsoptik eine Beleuchtungspupille innerhalb der Längserstreckung des Spiegels abbildet. Somit kann je nach dem an welchem Ort innerhalb der Längserstreckung die Beleuchtungspupille abgebildet wird ein gewünschter Beleuchtungswinkel insbesondere auf ein Patientenauge erhalten werden, beispielsweise eine +/-2°-Beleuchtung um eine homogenen roten Reflex zu erhalten. Ebenso kann eine 6°-Beleuchtung erhalten werden, wenn kein roter Reflex erforderlich ist.

Vorzugsweise wird eine derartige Beleuchtungseinrichtung an einem ophthalmologischen Operationsmikroskop eingesetzt. Sie kann aber auch bei Labormikroskopen, Technoskopen, Stereomikroskopen oder sonstigen Operationsmikroskopen in anderen Disziplinen eingesetzt werden. Auch ist es für den Fachmann ersichtlich, dass ein Operationsmikroskop, an dem eine derartige Beleuchtungseinrichtung angeordnet ist, über einen, zwei oder mehrere insbesondere auch stereoskopische Beobachtungsstrahlengänge verfügen kann. Beispielsweise ist ein Hauptmikroskop für den Operateur sowie ein Assistentenmikroskop vorgesehen.

Der grundsätzliche Aufbau eines Operationsmikroskops mit einer zugeordneten Beleuchtungseinrichtung kann unter anderem der EP 1 997 423 B1 oder der DE 10 2004 050 651 A1 entnommen werden, auf deren Offenbarungsgehalt hierzu ausdrücklich Bezug genommen wird.

Vorteilhafte Ausgestaltungen der Erfindung sind der Gegenstand von Unteransprüchen.

In einer bevorzugten Ausführungsform wird als Lichtquelle nicht mehr nur eine einzige Lichtquelle wie beispielsweise eine Halogenlampe eingesetzt beziehungsweise zwei räumlich getrennte Lichtquellen für unterschiedliche Beleuchtungswinkel sondern nur noch eine einzige Lichtquelle, die mehrere Licht emittierende Flächen aufweist, die dann jeweils die vorstehend genannten Flächenbereiche bilden. Dabei ist die Lichtquelle derart ausgelegt, dass diese mehreren Flächen unabhängig voneinander aktivierbar sind. Das bedeutet, dass beispielsweise eine, zwei, drei oder vier Flächen gemeinsam oder unabhängig voneinander Licht aussenden können. Auch ist bevorzugt, dass die Leistung der Licht emittierenden Flächen unabhängig voneinander ansteuerbar ist, um unterschiedliche Beleuchtungswinkel zu erhalten. Bevorzugt ist jede der einzelnen Licht emittierenden Flächen unabhängig voneinander regel- und/oder steuerbar. Das heißt sie können einzeln, gruppenweise oder alle zusammen ein- und ausgeschaltet oder gedimmt werden. Prinzipiell kann die Lichtquelle auch mehr Licht emittierende Flächen aufweisen. Insbesondere bei einer nachfolgend beschriebenen Ausgestaltung als Multichipemitter-LED ist es aufgrund der Anordnung der leuchtenden Flächen bzw. der einzelnen Segmente der LED möglich, eine Beleuchtung in gewünschter Art und Weise zu realisieren, also beispielsweise eine 2°-Beleuchtung oder eine 6°-Beleuchtung. Dies ergibt sich daraus, dass in Abhängigkeit der Anordnung der Lichtquelle bzw. deren Licht emittierenden Flächen die Ausgestaltung einer Beleuchtungsoptik derart gewählt werden kann, dass je nach Aktivierung einer oder mehrerer Flächen beispielsweise eine 2°- oder eine 6°-Beleuchtung realisierbar ist. Werden beispielsweise ein oder zwei Licht emittierende Flächen oberhalb der optischen Achse der Beleuchtungsoptik aktiviert wird ein anderer Winkel des Beleuchtungsstrahlengangs erhalten als wenn zwei Licht emittierende Flächen unterhalb der optischen Achse aktiviert werden. Eine entsprechende Ausgestaltung der Beleuchtungsoptik erfolgt anhand der Dimensionierung und Art der Lichtquelle sowie den Abmessungen und Eigenschaften beispielsweise des Operationsmikroskops. Zur Realisierung einer Lichtquelle mit mehreren Licht emittierenden Flächen können beispielsweise LEDs, Laser, durch Laser angeregte Konverter, Lichtleiter, DMDs (englisch: digital micromirror device, matrixförmig angeordnete Mikrospiegelaktoren) und dergleichen verwendet werden. Denkbar ist auch, dass beispielsweise mehrere einzelne LEDs oder sonstige Licht emittierende Flächen/Objekte nahe beieinander angeordnet sind und eine einzige Lichtquelle bilden. Dies führt jedoch zu einer größeren Baugröße insbesondere der Beleuchtungsoptik der Beleuchtungseinrichtung.

In bevorzugter Weise ist die Lichtquelle der Beleuchtungseinrichtung eine Multichipemitter-LED wie sie unter den Bezeichnungen Cree MCE oder SEOUL P4 derzeit erhältlich sind. Diese LEDs verfügen über vier Licht emittierende Flächen bzw. Segmente, die unabhängig voneinander ansteuerbar oder aktivierbar sind, um je nach aktivierter Licht emittierender Fläche einen anderen Beleuchtungswinkel zu erhalten. Eine solche LED hat beispielsweise vier Chips, die die vier Licht emittierenden Flächen bilden mit einer Kantenlänge von jeweils ca. 1 - 4mm.

Vorzugsweise können durch das selektive Aktivieren oder Ansteuern der Licht emittierenden Flächen der Lichtquelle eine an sich bekannte 2°- bzw. 6°-Beleuchtung mit den genannten Beleuchtungswinkeln realisiert werden, um beispielsweise ein zu untersuchendes Auge eines Patienten in gewünschter Weise ausleuchten zu können. Hierzu kann die Beleuchtungsoptik entsprechend ausgelegt sein, um je nach Einfallswinkel von einer der Licht emittierenden Flächen einen gewünschten Beleuchtungswinkel zu erhalten.

Weiterhin ist vorgeschlagen, dass die verschiedenen Licht emittierenden Flächen der Lichtquelle jeweils unterschiedliche Farben und/oder Helligkeiten aufweisen können. Beispielsweise kann eine Licht emittierende Fläche einen höheren Rotanteil aufweisen, um insbesondere den gewünschten Redreflex im Auge eines Patienten zu erzeugen. Eine andere Licht emittierende Fläche kann demgegenüber einen höheren Blauanteil im emittierten Spektrum aufweisen, um eine kaltweiße Umgebungsbeleuchtung des Auges zu erzeugen. Durch die veränderbaren Helligkeiten kann beispielsweise eine Belastung der Makula reduziert werden.

Zur Ausgestaltung der Beleuchtungseinrichtung ist vorgeschlagen, dass mindestens einer, vorzugsweise zwei oder mehr, insbesondere drei, Spiegel vorgesehen sind, wobei je nach Anzahl und Anordnung der aktivierten Licht emittierenden Flächen beziehungsweise der Licht emittierenden Flächenbereiche der Lichtquelle ein oder mehrere Spiegel beleuchtet werden. Besonders bei Systemen mit einem Assistentenmikroskop sind zwei Spiegel von Vorteil. Prinzipiell können auch geteilte und/oder durchbohrte und/oder teilbeschichtete Spiegel und/oder Strahlteiler verwendet werden. Bei dieser Ausgestaltung wird die Beleuchtungspupille innerhalb der Längserstreckung der Projektionen der Spiegel auf die optische Achse abgebildet. Dabei muss die Beleuchtungspupille nicht notwendiger Weise unmittelbar auf einen Spiegel selbst abgebildet werden sondern kann, entlang der optischen Achse gesehen auch zwischen zwei Spiegel abgebildet werden.

Weiterhin ist vorgeschlagen, dass die Lichtquelle selbst entlang der optischen Achse verlagerbar ist, um das von ihr ausgehende Licht beziehungsweise die Beleuchtungspupille durch die Beleuchtungsoptik hindurch an einem gewünschten Ort entlang der optischen Achse zu projizieren.

Schließlich ist vorgeschlagen, dass die Lichtquelle zumindest drei Licht emittierende Flächen aufweist, die senkrecht zur optischen Achse verteilt angeordnet sind, insbesondere in zwei Richtungen versetzt zueinander. Somit kann jeder Lichtquelle beispielsweise genau ein Spiegel zugeordnet werden, um eine Beleuchtung des Objekts gewünschter Art zu erhalten.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ein Ausführungsbeispiel der Erfindung ist in der Zeichnung dargestellt und wird in der nachfolgenden Beschreibung näher erläutert. Es zeigt:

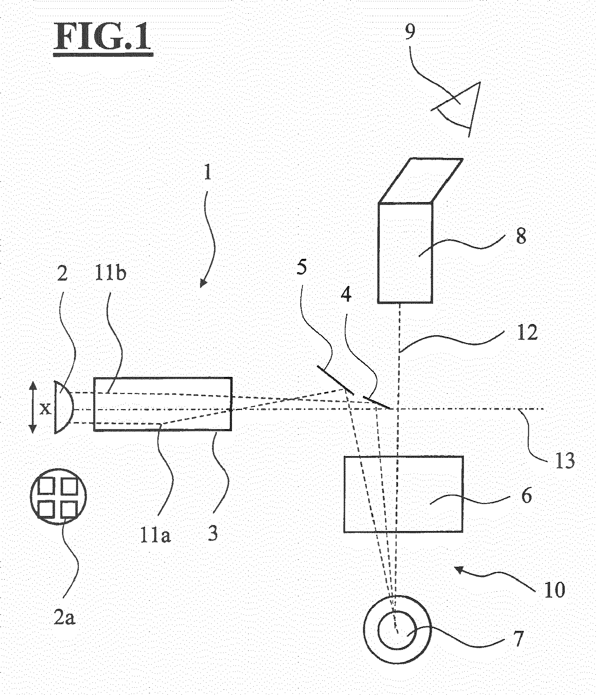

- Figur 1 bis 6: verschiedene Operationsmikroskope in schematischer Darstellung.

Bei dem in Figur 1 dargestellten Ausführungsbeispiel handelt es sich um ein Operationsmikroskop 10 mit einer Beleuchtungseinrichtung 1, um beispielsweise eine Kataraktoperation an einem Patientenauge 7 vorzunehmen. Die Beleuchtungseinrichtung 1 umfasst eine Multichipemitter-LED 2 mit vier Licht emittierenden Flächen 2a, wie durch die Darstellung unterhalb der LED 2 angedeutet. Größe und Formgebung der Licht emittierenden Flächen 2a ist an sich beliebig gestaltbar beispielsweise jeweils als Kreisausschnitt. Um das Licht der Multichipemitter LED 2 zu dem eigentlichen Operationsmikroskop 10 zu leiten dient eine hier nur schematisch angedeutete Beleuchtungsoptik 3 mit einer strichpunktiert eingezeichneten optischen Achse 13, die beispielsweise zwei Linsen sowie eine oder mehrere Blenden umfassen kann und das von der LED 2 ausgehende Licht in gewünschter Weise formt und beispielsweise eine Köhlersche Optik bildet. In diesem Fall ist die Beleuchtungspupille identisch mit dem Bild der Lichtquelle 2. Zur Umlenkung dienen hier zwei Spiegel 4, 5, die gegebenenfalls auch halb durchlässig ausgebildet sein können, um das Licht durch eine weitere Abbildungsoptik 6, die hier ebenfalls nur schematisch dargestellt ist, auf ein Objektfeld insbesondere das Auge 7 eines zu untersuchenden oder zu operierenden Patienten zu leiten. Zur Beobachtung dient eine Beobachtungsoptik 8, durch die ein Auge 9 eines Betrachters das Objektfeld beziehungsweise das Auge 7 einsehen kann. Der grundsätzliche Aufbau beispielsweise der Beobachtungsoptik 8, der Abbildungsoptik 6 sowie weiterer Komponenten eines Operationsmikroskops 10 kann beispielsweise den vorstehend genannten Druckschriften entnommen werden.

Wie durch die zwei gestrichelten Beleuchtungsstrahlengänge 11a, 11b von der LED 2 ausgehend angedeutet erfolgt eine unterschiedliche Ausleuchtung des zu untersuchenden Objektfelds oder Auges 7 je nachdem welche Licht emittierenden Flächen 2a aktiviert sind. Die Beleuchtungsstrahlengänge 11a, 11b sind stellvertretend für die entsprechenden Lichtbüschel eingezeichnet. Dabei wird beispielsweise durch Aktivierung von zwei Licht emittierenden Flächen 2a oberhalb der optischen Achse 13 der Beleuchtungsstrahlengang 11a und durch Aktivierung von zwei Licht emittierenden Flächen 2a unterhalb der optischen Achse 13 der Beleuchtungsstrahlengang 11b erhalten. Somit wird der Licht emittierende Flächenbereich der Lichtquelle senkrecht zur optischen Achse 13 variiert, wie durch den Doppelpfeil x angedeutet. Es ist klar, dass alternativ oder zusätzlich eine Variation des Licht emittierenden Flächenbereichs auch in einer Richtung senkrecht zur Zeichenebene variiert werden kann. Dabei ist es für den Fachmann ersichtlich, dass ein realer Beleuchtungsstrahlengang 11 die Beleuchtungsoptik 3 auf einem anderen optischen Weg durchläuft als es hier nur schematisch angedeutet ist. Nicht massstäblich sind hier unterschiedliche Beleuchtungswinkel für die von unterschiedlichen Licht emittierenden Flächen 2a ausgehenden Beleuchtungsstrahlengänge 11a, 11b dargestellt. So kann beispielsweise eine 2°- oder eine 6°-Beleuchtung, d.h. schräg zu einem Beobachtungsstrahlengang 12, der hier ebenfalls gestrichelt angedeutet ist, realisiert werden. Weiterhin verfügt die Beleuchtungseinrichtung 1 über keinerlei bewegliche Bauteile, so dass praktisch kein Verschleiß auftritt. Auch kann spezifisch nur derjenige Beleuchtungsstrahlengang 11a, b erzeugt werden, der tatsächlich gewünscht ist. Es wird kein unerwünschtes Licht erzeugt, das nachträglich wieder abgeblendet oder in einer Lichtfalle ausgelöscht werden müsste.

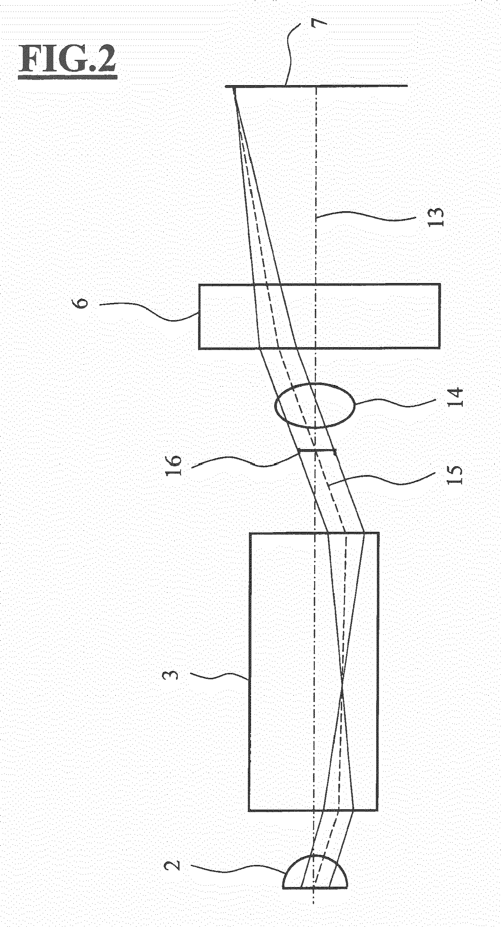

In Figur 2 ist als Beispiel die Beleuchtungspupille 16 einer Köhler-Beleuchtung dargestellt. Das Licht der Lichtquelle 2 wird durch die Beleuchtungsoptik 3 und die Abbildungsoptik 6 auf das Objektfeld beziehungsweise das Auge 7 fokussiert, wobei das Lichtbüschel 14 mit Hauptstrahl 15 den Rand des Auges 7 beleuchtet. Der Hauptstrahl 15 schneidet die optische Achse 13 in der Beleuchtungspupille 16. Diese Definition der Beleuchtungspupille 16 gilt für alle Beleuchtungsprinzipien, nicht nur für die Köhler-Beleuchtung. Im Fall der Köhler-Beleuchtung ist die Beleuchtungspupille 16 identisch mit dem Bild der Lichtquelle 2.

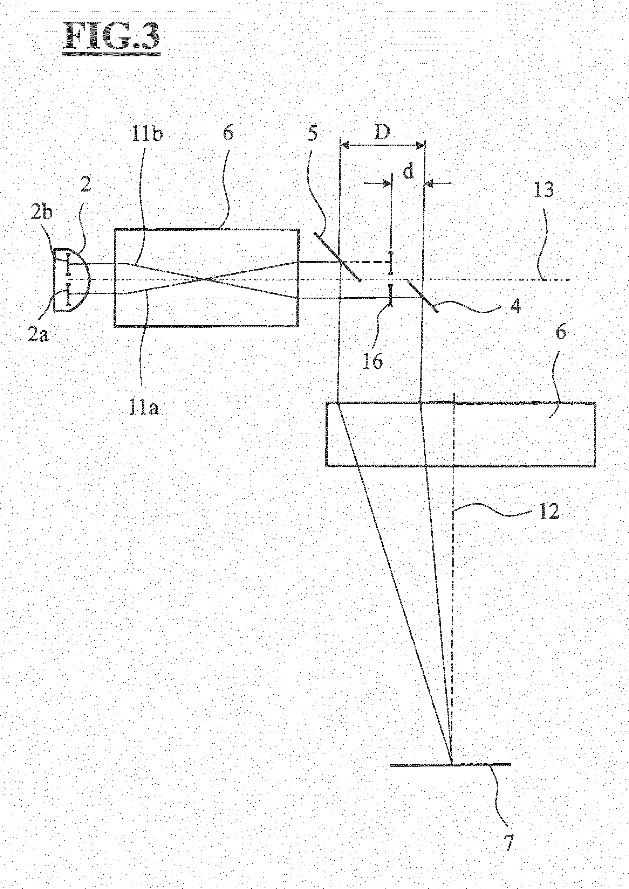

Wegen der besseren Übersichtlichkeit sind die Spiegel 4 und 5 weggelassen; sie werden vorzugsweise vor beziehungsweise hinter der Beleuchtungspupille angeordnet, wie in Fig.3 dargestellt.

In Figur 3 besteht die Lichtquelle 2 aus zwei Licht emittierenden Flächen 2a, 2b die in die Beleuchtungspupille 16 abgebildet werden. Dargestellt ist hier wieder der Fall der Köhler-Beleuchtung.

Stellvertretend für die Beleuchtungssstrahlenbüschel sind die Beleuchtungsstrahlengänge 11a und 11b eingezeichnet, die das Objektfeld 7 unter verschiedenen Winkeln beleuchten. Der Beleuchtungsstrahlengang 11a erzeugt eine 6°-Beleuchtung, der Beleuchtungsstrahlengang 11b erzeugt eine +2°-Beleuchtung. Zur Verdeutlichung ist auch der Beobachtungsstrahlengang 12 eingezeichnet.

Die Beleuchtungspupille 16 liegt zwischen den Spiegeln 4 und 5, vorzugsweise gilt:
d ist gleich 0 bis 0,8 D
mit:
d = Abstand Beleuchtungspupille 16 - Spiegel 4
D = Abstand der Spiegelmitten

Der Spiegel 4 ist der Spiegel mit der kleineren Fläche wobei die Spiegel 4 und 5 auch vertauscht sein können. Dabei ist die Position der Spiegel 4, 5 jeweils mit ihrer Mitte, gesehen entlang ihrer Erstreckung bezüglich der optischen Achse 13, anzusehen.

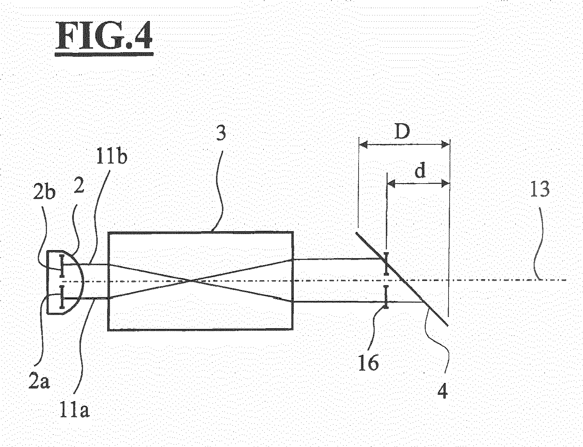

In Figur 4 besteht die Lichtquelle 2 aus zwei Licht emittierenden Flächen 2a, 2b die in die Beleuchtungspupille 16 abgebildet werden. Dargestellt ist hier wieder der Fall der Köhler-Beleuchtung.

Stellvertretend für die Beleuchtungssstrahlenbüschel sind die Beleuchtungsstrahlengänge 11a und 11b eingezeichnet, die das Objektfeld 7 unter verschiedenen Winkeln beleuchten. Der Beleuchtungsstrahlengang 11b erzeugt eine 6°-Beleuchtung, der Beleuchtungsstrahlengang 11a erzeugt eine +2°-Beleuchtung. Zur Vereinfachung der Darstellung ist die an sich bekannte Abbildungsoptik 6 sowie das Objektfeld 7 weggelassen.

In Figur 4 ist die Ausgestaltung mit nur einem einzigen Spiegel 4 abgebildet, wobei hier die Beleuchtungsoptik 3 derart ausgebildet ist, dass die Beleuchtungspupille 16 innerhalb der Längserstreckung der Projektion des Spiegels 4 auf die optische Achse 13 abgebildet wird. In diesem Fall werden die Werte vorzugsweise wie folgt gewählt:
d = 0.2 D bis 0.8 D
mit:
d = Abstand Beleuchtungspupille 16 - hinterer Spiegelrand, entlang der optischen Achse 13 gesehen
D = Längserstreckung des Spiegels 4 längs der optischen Achse 13.

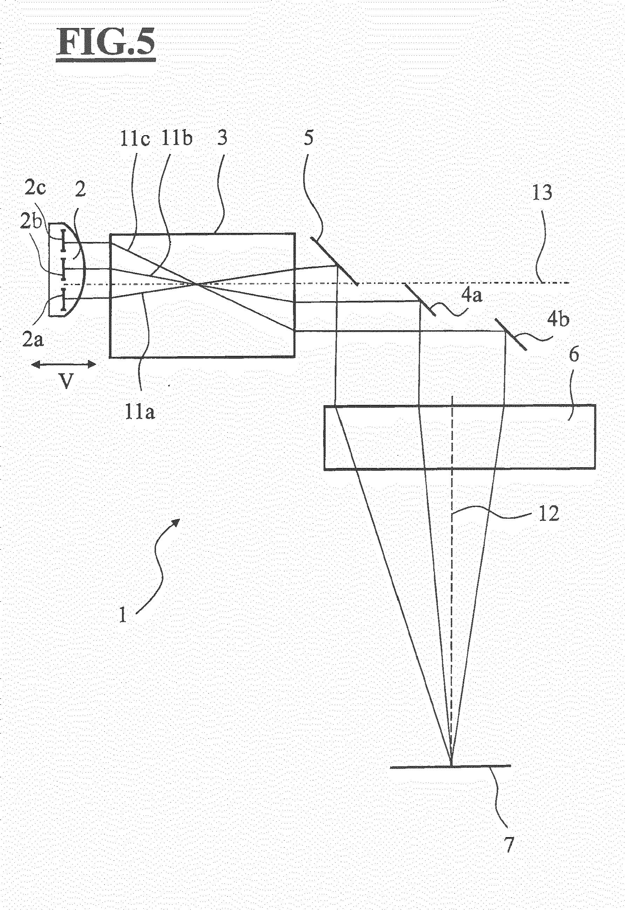

In Figur 5 ist eine Beleuchtungseinrichtung 1 mit einer Lichtquelle 2 mit drei Licht emittierenden Flächen 2a, b, c abgebildet, die wie aus der Darstellung ersichtlich und durch den Doppelpfeil V angedeutet, beispielsweise mechanisch entlang der optischen Achse 13 verschiebbar ist. Durch die Ausgestaltung der Beleuchtungsoptik 3 werden die Beleuchtungsstrahlengänge 11a, 11b, 11c derart auf die drei Spiegel 4a, 4b, 5 projiziert, dass eine +/-2° Beleuchtung und eine 6°-Beleuchtung erhalten werden kann je nach dem welche Licht emittierende Fläche 2a gerade aktiviert ist. Der Beleuchtungsstrahlengang 11a erzeugt eine 6°-Beleuchtung, der Beleuchtungsstrahlengang 11b erzeugt eine +2°-Beleuchtung und der Beleuchtungsstrahlengang 11c erzeugt eine -2°-Beleuchtung. Zur Verdeutlichung ist auch der Beobachtungsstrahlengang 12 eingezeichnet.

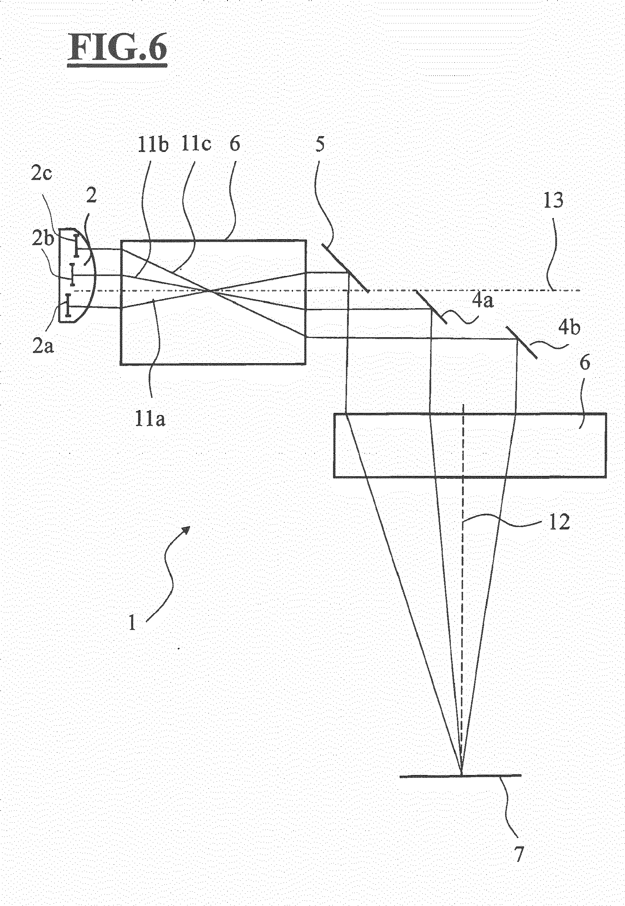

In Figur 6 ist eine weitere Beleuchtungseinrichtung 1 abgebildet. Diese umfasst eine Lichtquelle 2 mit drei Licht emittierenden Flächen 2a, 2b, 2c, die entlang der optischen Achse 13 zueinander versetzt angeordnet sind. Die von den Licht emittierenden Flächen 2a, 2b, 2c ausgehenden Beleuchtungsstrahlengänge 11a, 11b, 11c werden durch die Beleuchtungsoptik 3 jeweils auf den Spiegeln 5, 4a, 4b abgebildet. Dabei ist der Versatz der Licht emittierenden Flächen 2a, 2b, 2c entlang der optischen Achse 13 derart gewählt und die Beleuchtungsoptik 3 derart ausgebildet, dass beispielsweise mit dem Beleuchtungsstrahlengang 11a eine 6°-Beleuchtung und mit den Beleuchtungsstrahlengängen 11b, 11c eine +2°- und -2°-Beleuchtung erzeugt werden kann, wenn die entsprechende Licht emittierende Fläche 2a, 2b, 2c aktiviert ist.

### Bezugszeichenliste:

- 1: Beleuchtungseinrichtung
- 2: Multichipemitter LED
- 2a: lichtemittierende Fläche
- 3: Beleuchtungsoptik
- 4: Spiegel
- 5: Spiegel
- 6: Abbildungsoptik
- 7: Patientenauge
- 8: Beobachtungsoptik
- 9: Betrachter
- 10: Operationsmikroskop
- 11: Beleuchtungsstrahlengang
- 12: Beobachtungsstrahlengang
- 13: optische Achse der Beleuchtungsoptik 3
- 14: Lichtbüschel
- 15: Hauptstrahl
- 16: Beleuchtungspupille

- x: Variation der Position der Lichtquelle 2
- y: Variation der Position der Lichtquelle 2 entlang der optischen Achse 13
- V: Verschiebung der Lichtquelle 2 entlang der optischen Achse 13

## Patentansprüche

1. Beleuchtungseinrichtung (1), insbesondere eines Operationsmikroskops (10), umfassend eine Beleuchtungsoptik (3) mit einer optischen Achse (13) und einer Lichtquelle (2) zum Erzeugen wenigstens eines Beleuchtungsstrahlengangs (11) zum Beleuchten eines Objektfelds unter einem bestimmten Beleuchtungswinkel und mit einem Beobachtungsstrahlengang (12), wobei im Beleuchtungsstrahlengang mindestens ein Spiegel (4, 5) zum Umlenken von Licht der Lichtquelle (2) vorgesehen ist und der Spiegel (4, 5) entlang der optischen Achse (13) eine Längserstreckung aufweist, wobei ein Licht emittierender Flächenbereich der Lichtquelle (2) ohne bewegliche Bauteile senkrecht zur optischen Achse (13) variierbar (x) ist,
**dadurch gekennzeichnet, dass**
die Beleuchtungsoptik (3) eine Beleuchtungspupille (16) innerhalb der Längserstreckung des Spiegels (4, 5) abbildet.

2. Beleuchtungseinrichtung (1) nach Anspruch 1, **dadurch gekennzeichnet, dass** die Lichtquelle (2) mehrere Licht emittierende Flächen (2a) aufweist, die unabhängig voneinander aktivierbar sind.

3. Beleuchtungseinrichtung (1) nach Anspruch 2, **dadurch gekennzeichnet, dass** die Lichtquelle eine Multichipemitter-LED (2) ist.

4. Beleuchtungseinrichtung (1) nach einem der Ansprüche 1 bis 3, **dadurch gekennzeichnet, dass** unterschiedliche Beleuchtungswinkel realisierbar sind, insbesondere 2° und 6°.

5. Beleuchtungseinrichtung (1) nach einem der Ansprüche 2 bis 4, **dadurch gekennzeichnet, dass** die Licht emittierenden Flächen (2a) unterschiedliche Farben und/oder Helligkeiten aufweisen.

6. Beleuchtungseinrichtung (1) nach einem der Ansprüche 1 bis 5, **dadurch gekennzeichnet, dass** zwei oder mehr, insbesondere drei, Beleuchtungsspiegel (4, 5) vorgesehen sind.

7. Beleuchtungseinrichtung (1) nach einem der Ansprüche 1 bis 6, **dadurch gekennzeichnet, dass** die Lichtquelle (2) selbst entlang der optischen Achse (13) verlagerbar ist.

8. Beleuchtungseinrichtung (1) nach einem der Ansprüche 1 bis 7, **dadurch gekennzeichnet, dass** die Lichtquelle (2) zumindest drei Licht emittierende Flächen (2a) aufweist, die senkrecht zur optischen Achse (13) verteilt angeordnet sind, insbesondere in zwei Richtungen versetzt zueinander.

9. Ophthalmologisches Operationsmikroskop (10), **dadurch gekennzeichnet, dass** es mit einer Beleuchtungseinrichtung (1) nach einem der Ansprüche 1 bis 8 ausgestattet ist.
